# EUROPEAN PATENT APPLICATION

(11) **EP 3 851 152 A1**
(43) Date of publication of application: **21.07.2021**
(21) Application number: 18933669.6
(22) Date of filing: 11.09.2018
(51) Int. Cl.: A61M 29/00, A61F 2/82

(54) **INDWELLING DEVICE**

(71) Applicant: ASAHI INTECC CO., LTD., Seto-shi, Aichi 489-0071 (JP)
(72) Inventor: SATO Toshihiko, Kyoto-shi, Kyoto 606-8501 (JP); YUTAKA Yojiro, Kyoto-shi, Kyoto 606-8501 (JP); AIBA Hiroyuki, Seto-shi, Aichi 489-0071 (JP); FUKUOKA Tomochika, Seto-shi, Aichi 489-0071 (JP)
(74) Representative: Winter, Brandl - Partnerschaft mbB
(86) International application number: PCT/JP2018/033572
(87) International publication number: WO 2020/053948

(57) **Abstract**

In an indwelling device 1 including a device body 3 and an engaging part 5 connected to the device body 3 and capable of abutting on a bodily wall 99, the engaging part 5 can abut on the bodily wall 99 in a state that a region of the engaging part 5, excluding end parts, is expanded larger than an outer diameter of the device body 3.

## Description

### TECHNICAL FIELD

The present invention relates to an indwelling device that is placed in a patient's body lumen, such as a bronchus of a lung and a blood vessel in a patient.

### BACKGROUND ART

In conventional surgery or the like, a technique, so-called RFID (radio frequency identifier) has been used, in which a wireless marker is embedded in or near a patient's lesion, and a position of the patient's lesion is specified by wireless communication between the wireless marker and a device outside of the patient's body.

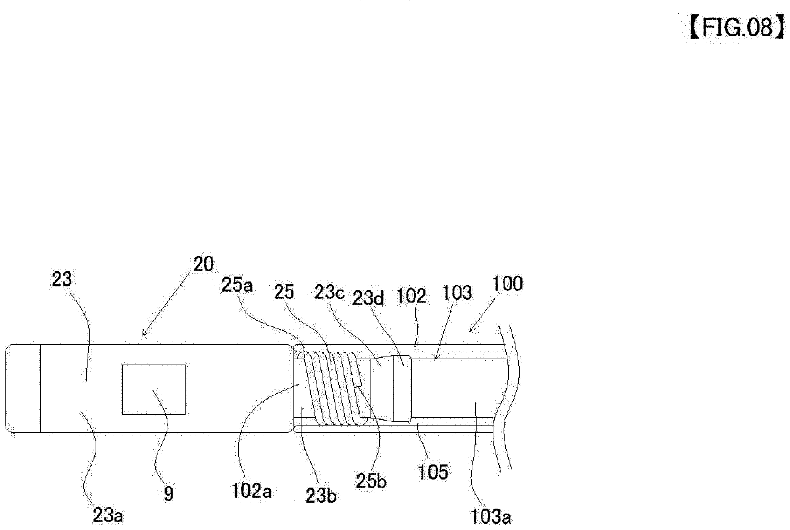

For example, Patent Document 1 describes that a wireless marker is embedded in a lesion in a bronchus of a patient's lung, and, during surgery or the like, a position of a lesion is informed to an operator in real time by wireless communication between a position-specifying system outside of the patient's body and the wireless marker (see FIG. 8 and the like).

In addition, the wireless marker used for this position-specifying system includes a leg (hereinafter referred to as "engagement part") as a mechanism for the wireless marker to be fixed inside the patient's body (see FIG. 13 and the like).

### CITATION LIST

### Patent Literature

Patent Literature 1: Japanese Unexamined Patent Application Publication No. 2013-543401

### SUMMARY OF INVENTION

### Problem to be Solved

However, the conventional wireless marker has had a problem that a hook-shaped proximal end part of an engaging part is likely to damage a patient's bodily wall.

In addition, the conventional wireless marker has had a problem that, due to a relationship between a shape of a wireless marker body and a shape of the engaging part, the wireless marker is likely to occlude a patient's body lumen when disposing the wireless marker in the patient's body lumen.

Also, the conventional wireless marker has had a problem that the wireless marker is difficult to insert into a catheter lumen and also difficult to carry thereafter because the engaging part is disposed so as to project from a proximal end of the wireless marker toward the proximal end side.

Furthermore, the conventional wireless marker has had a problem that, since the engaging part is disposed on the proximal end part of the wireless marker, it is not until the whole wireless marker leaves from a distal end of a catheter for conveying the wireless marker that the wireless marker engages with the patient's body lumen, and therefore the wireless marker cannot be swiftly disposed in the patient's body lumen.

The present invention has been made in response to the aforementioned problems of the prior art, and objects of the present invention is to provide an indwelling device that does not occlude an inside of a body and does not damage a patient's bodily wall, and to provide an indwelling device that can be easily accommodated in a lumen such as a catheter and improves deliverability of the indwelling device, and further to provide an indwelling device that has an engaging part capable of swiftly abutting on the bodily wall and consequently can be swiftly placed in the body.

### Solution to Problem

In order to solve the aforementioned problems, the first aspect of the present invention is characterized in that, in an indwelling device including a device body and an engaging part connected to the device body and capable of abutting on a bodily wall, the engaging part can abut on the bodily wall in a state that a region of the engaging part, excluding end parts, is expanded larger than an outer diameter of the device body.

In addition, the second aspect of the present invention is characterized in that, in the indwelling device according to the first aspect, the device body has a small-diameter part having an outer diameter smaller than of other regions, and the engaging part can be stored on the small-diameter part.

In addition, the third aspect of the present invention is characterized in that, in the indwelling device according to the first or second aspect, the engaging part is constructed by spirally winding one or more wires.

Furthermore, the fourth aspect of the present invention is characterized in that, in the indwelling device according to the third aspect, an end part of the wire on a side not connected to the device body is located on a distal end side in an advancing direction of the indwelling device while being placed in a body.

### Effect of the Invention

According to the first aspect of the present invention, in the indwelling device including the device body and the engaging part connected to the device body and capable of abutting on the bodily wall, the engaging part can abut on the bodily wall in a state that the region of the engaging part excluding the end parts is expanded larger than the outer diameter of the device body, and therefore the indwelling device can be securely placed in the body without occluding the inside of the body and damaging the patient's bodily wall.

In addition, according to the second aspect of the present invention, in the indwelling device according to the first aspect, the device body has the small-diameter part having the outer diameter smaller than of other regions, and the engaging part can be stored on the small-diameter part, so that not only is the effect of the indwelling device according to the first aspect obtained but the indwelling device also becomes easier to accommodate in a lumen such as a catheter, and deliverability of the indwelling device can be improved.

In addition, according to the third aspect of the present invention, in the indwelling device according to the first or second aspect, the engaging part is constructed by spirally winding one or more wires, and therefore not only is the effect of the indwelling device according to the first or second aspect obtained but an engaging part that exhibits such an effect can also be easily constructed.

Furthermore, according to the fourth aspect of the present invention, in the indwelling device according to the third aspect, the end part of the wire on the side not connected to the device body is located on the distal end side in the advancing direction of the indwelling device while being placed in a body, and therefore not only is the effect of the indwelling device according to the third aspect obtained but the engaging part can also be swiftly abutted on the bodily wall, and consequently the indwelling device can be swiftly placed in the body.

### BRIEF DESCRIPTION OF DRAWINGS

FIG. 1 is a schematic overall view of a medical appliance for placing an indwelling device according to the first embodiment of the present invention in a body.
FIG. 2 is a longitudinal sectional view of FIG. 1.
FIG. 3 is a longitudinal sectional view of the medical appliance, illustrating a state that the indwelling device according to the first embodiment is pushed out from the medical appliance.
FIG. 4 is an enlarged sectional view of a part A in FIG. 2.
FIG. 5 is an enlarged sectional view of a part B in FIG. 3.
FIG. 6 is an enlarged sectional view corresponding to FIG. 4 in a case that an indwelling device according to the second embodiment is disposed.
FIG. 7 is an enlarged sectional view corresponding to FIG. 5 in a case that an indwelling device according to the second embodiment is disposed.
FIG. 8 is an overall view of an indwelling device according to the third embodiment.
FIG. 9 is an overall view of an indwelling device according to the fourth embodiment.
FIG. 10 is an overall view of an indwelling device according to the fifth embodiment.
FIG. 11 is an overall view of an indwelling device according to the sixth embodiment.
FIG. 12 is an overall view of an indwelling device according to the seventh embodiment.

### EMBODIMENTS OF THE INVENTION

Hereinafter, embodiments of the present invention will be explained with reference to the drawings.

### (First Embodiment)

First, the first embodiment of the present invention will be explained.

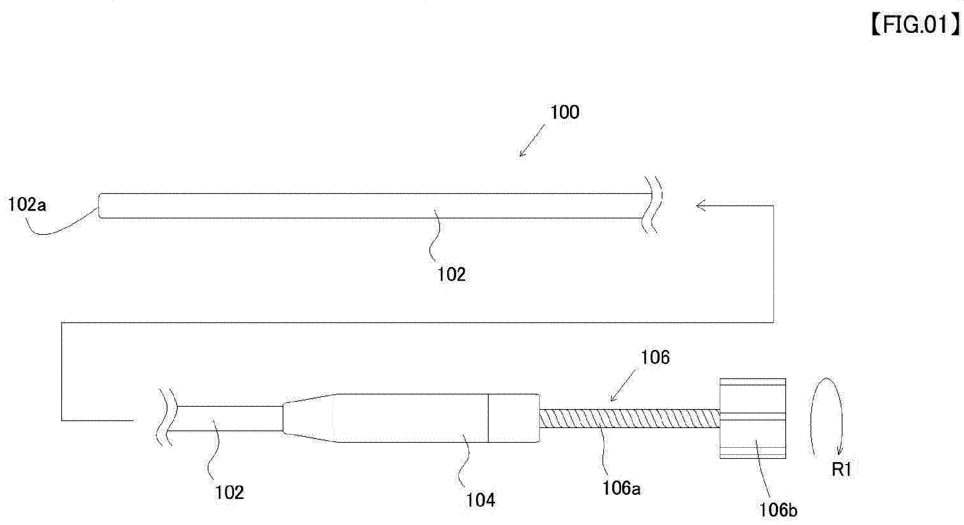

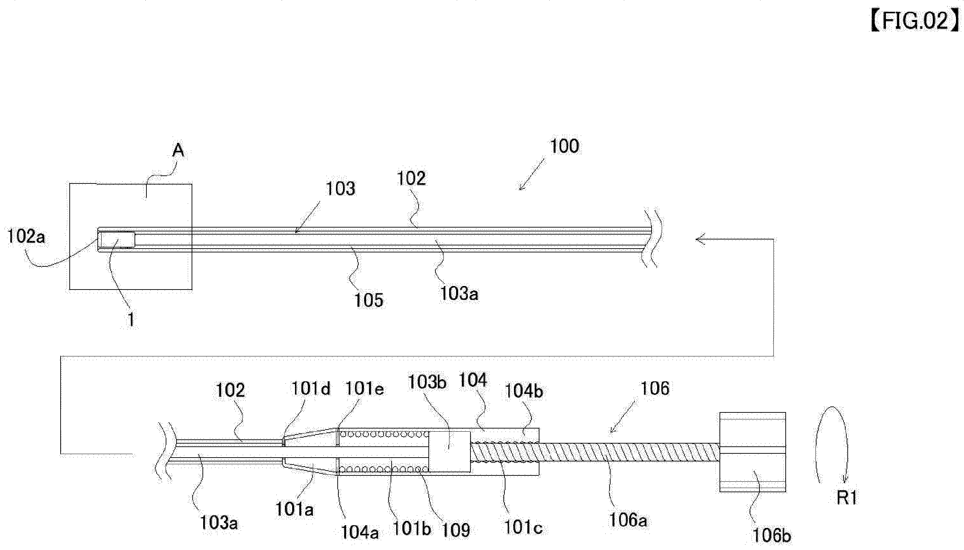

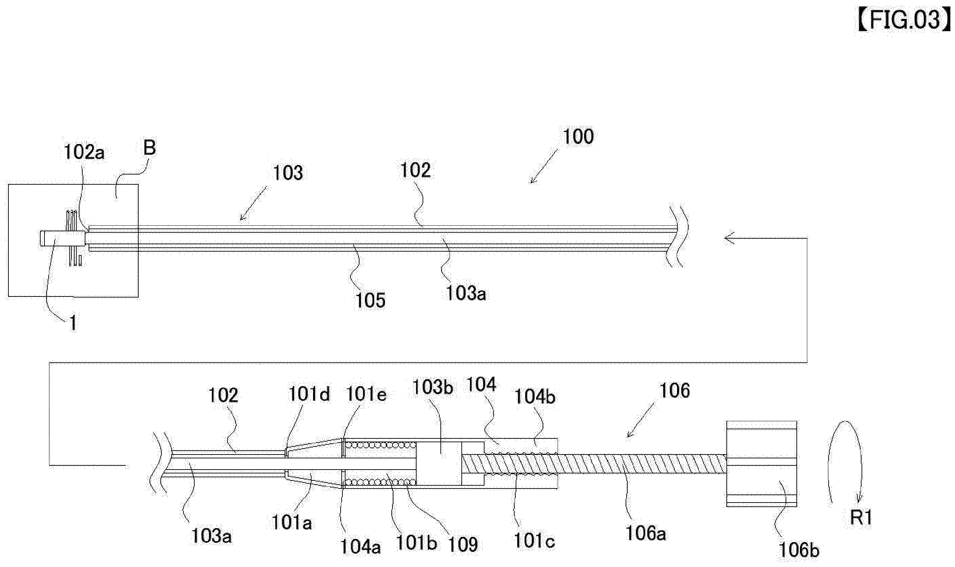

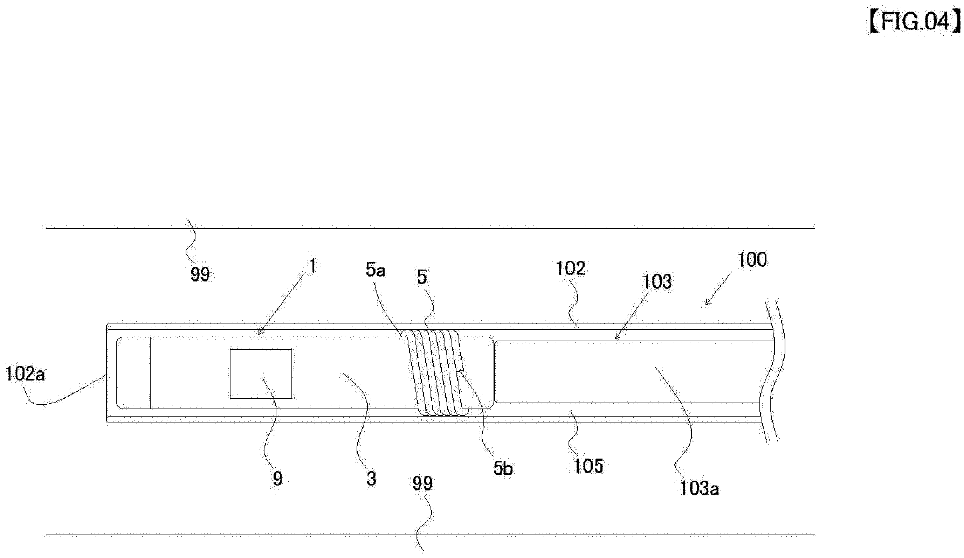

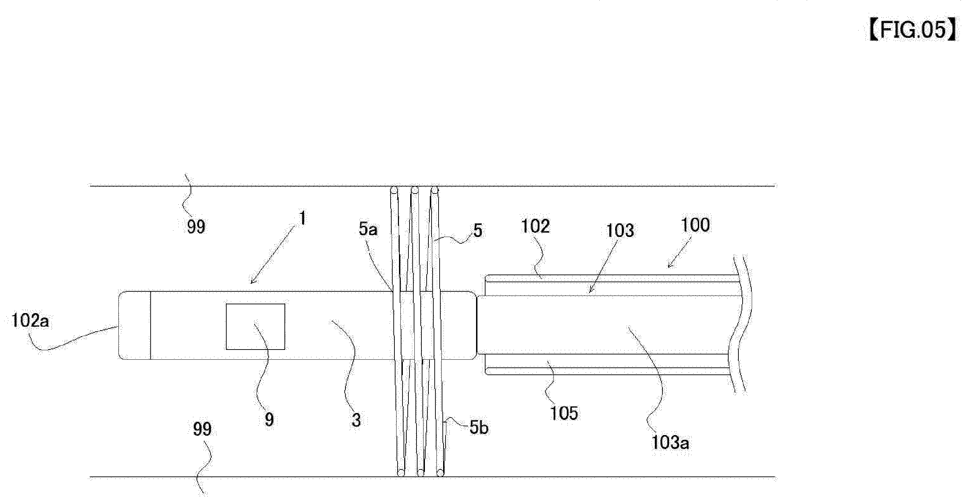

FIG. 1 is a schematic overall view of a medical appliance for placing an indwelling device according to the first embodiment of the present invention in a body. FIG. 2 is a longitudinal sectional view of FIG. 1. FIG. 3 is a longitudinal sectional view of the medical appliance, illustrating a state that the indwelling device according to the first embodiment is pushed out from the medical appliance. FIG. 4 is an enlarged sectional view of a part A in FIG. 2. FIG. 5 is an enlarged sectional view of a part B in FIG. 3.

As illustrated in FIG. 1 to FIG. 3, a medical appliance 100 for placing an indwelling device 1 (see FIG. 2 to FIG. 5) according to the first embodiment described below in a patient's body is composed of a catheter body 102, a connector 104 connected to a proximal end of the catheter body 102, a pusher member 103 slidably inserted into a catheter lumen 105 of the catheter body 102 as well as a distal end-side lumen 101a and an intermediate lumen 101b of the connector 104, and a grip part 106 abutting on a proximal end of the pusher member 103.

The catheter body 102 is a hollow cylindrical flexible long member and has the catheter lumen 105 inside thereof and an opening 102a on the distal end thereof.

A material for the catheter body 102 is a resin material made of a biocompatible material. Various synthetic resin materials, for example, a polyolefin resin such as a polyamide resin, a polyamide elastomer, a polyester, a polyurethane, a polyvinyl chloride resin, a polyethylene, a polypropylene, and an ethylene-propylene copolymer, a fluororesin such as Teflon (registered trademark), an ethylene-vinyl acetate copolymer can be used, and in the first embodiment, a polyamide elastomer is used.

The pusher member 103 is composed of a solid cylindrical flexible long member 103a and an abutting part 103b connected to the proximal end of the long member 103a. The pusher member 103 is slidably inserted into the catheter lumen 105 of the catheter body 102, a distal end of the pusher member 103 is located in the vicinity of a distal end of the catheter body 102, and the proximal end of the pusher member 103 is located inside of the connector 104.

Incidentally, a material for the flexible long member 103a of the pusher member 103 is a metal material made of a biocompatible material, and for example, various metal materials such as a stainless steel, an NiTi shape - memory alloy, and a cobalt-chromium alloy can be used, and in the first embodiment, a stainless steel is used.

In addition, a material for the abutting part 103b of the pusher member 103 is not particularly limited, and an ordinary resin material such as polyacetal and polycarbonate can be used.

In the grip part 106, a screw part 106a located on the distal end and a grip part body 106b connected to a proximal end of the screw part 106a and having an outer diameter larger than of the screw part 106a are integrally formed.

A material for the grip part 106 is not particularly limited as long as the material has a higher rigidity than of the catheter body 102. For example, a polyethylene, a polypropylene, a polycarbonate, a polyacetal, a polyvinyl chloride (PVC), a polybutadiene (PBD), an ABS resin, or the like can be used, and in the first embodiment, a polyvinyl chloride (PVC) is used.

The connector 104 is a hollow member having a tapered distal end side and a hollow cylindrical proximal end side, and the distal end is connected to the proximal end of the catheter body 102. The connector 104 has the distal end-side lumen 101a that communicates with the catheter lumen 105 of the catheter body 102 through a circular opening 101d located on the distal end of the connector 104, the intermediate lumen 101b located on a middle part of the connector 104, and a proximal end-side lumen 101c located on the proximal end side of the connector 104.

In addition, the connector 104 has an intermediate wall 104a between the distal end-side lumen 101a and the intermediate lumen 101b, and a circular opening 101e is formed in the center of the intermediate wall 104a. In addition, a spiral screw groove 104b is formed on an inner surface of the connector 104, where the proximal end-side lumen 101c of the connector 104 is formed.

The screw part 106a of the grip part 106 is screwed into the screw groove 104b of the connector 104. Thus, when the grip part body 106b is turned in a R1 direction (clockwise direction toward the distal direction) with respect to the connector 104, the grip part 106 moves to the left side of the figure with respect to the connector 104.

A proximal end of the flexible long member 103a of the pusher member 103 is inserted through the opening 101d and the opening 101e of the connector 104 to the intermediate lumen 101b of the connector 104, and the abutting part 103b located on the proximal end of the pusher member 103 is disposed inside of the intermediate lumen 101b of the connector 104.

Herein, in the intermediate lumen 101b of the connector 104, a compression spring 109 is disposed between the intermediate wall 104a and the abutting part 103b of the pusher member 103, and the compression spring 109 always acts so as to energize the abutting part 103b toward the right side of the figure.

Thus, when the grip part body 106b is turned in the R1 direction with respect to the connector 104, the grip part 106 moves to the left side of the figure with respect to the connector 104 against an energizing force of the compression spring 109 for energizing the abutting part 103b toward the right side of the figure, and at the same time, the pusher member 103 also moves to the left side of the figure.

On the other hand, when the grip part body 106b is turned in an inverse R1 direction with respect to the connector 104, the abutting part 103b is energized toward the right side of the figure by the compression spring 109, so that the grip part 106 moves to the right side of the figure with respect to the connector 104, and at the same time, the pusher member 103 also moves to the right side of the figure.

Also, a material for the connector 104 is not particularly limited as long as the material has a higher rigidity than of the catheter body 102. For example, a polyethylene, a polypropylene, a polycarbonate, a polyacetal, a polyvinyl chloride (PVC), a polybutadiene (PBD), an ABS resin, or the like can be used, and in the first embodiment, a polyvinyl chloride (PVC) is used.

Herein, the indwelling device 1 according to the first embodiment will be explained. The indwelling device 1 is explained in such a way that the advancing direction of the medical appliance 100 i.e. a direction toward a patient's lesion is a distal end side and an opposite side thereto is a proximal end side.

As illustrated in FIG. 4, the indwelling device 1 includes an IC tag 9 that stores various information in an inside thereof. The indwelling device 1 is composed of the device body 3, and the engaging part 5 connected to the proximal end side of the device body 3.

The device body 3 according to the first embodiment is a hollow cylindrical member having a diameter of several millimeters. As a material for the device body 3, a resin material made of a biocompatible material, e.g. various synthetic resin materials including a polyolefin resin such as a polyamide resin, a polyamide elastomer, a polyester, a polyurethane, a polyvinyl chloride resin, a polyethylene, a polypropylene and an ethylene-propylene copolymer, a fluororesin such as Teflon (registered trademark), an ethylene-vinyl acetate copolymer, and the like; as well as a metal material made of a biocompatible material can be used.

The engaging part 5 according to the first embodiment is composed of a coil constructed by winding one NiTi shape-memory alloy wire in a hollow shape, and the engaging part 5 is wound around a proximal outer periphery of the device body 3 in a state that the coil is contracted. Incidentally, in the engaging part 5 according to the first embodiment, a distal end part 5a of the coil is fixed to the device body, and a proximal end part 5b of the coil is a free end.

Incidentally, since the engaging part 5 according to the first embodiment is simply constructed by winding one NiTi shape-memory alloy wire in a hollow shape, the engaging part that exerts an effect described below in the first embodiment can be easily constructed.

The indwelling device 1 explained above is inserted into the catheter lumen 105 on the distal end of the catheter body 102, and the distal end of the pusher member 103 is abutted on the proximal end of the indwelling device 1. Then, the catheter body 102 in that state is inserted into the patient's body and advanced to the vicinity of the patient's lesion. FIG. 2 and FIG. 4 illustrate that state.

In addition to FIG. 2 and FIG. 3, FIG. 4 and FIG. 5 illustrate the bodily wall 99 in the patient's lesion for the purpose of explaining an engaging state of the indwelling device.

Then, when the grip part body 106b is turned in the R1 direction with respect to the connector 104, the grip part 106 moves to the left side of the figure with respect to the connector 104, and at the same time, the pusher member 103 also moves to the left side of the figure, the indwelling device 1 is pushed out from the catheter body 102 as illustrated in FIG. 3 and FIG. 5, and the engaging part 5 composed of the coil is expanded, so that the engaging part 5 abuts on and engages with the bodily wall 99 in a state that a region of the engaging part 5 excluding the end parts is expanded larger than an outer diameter of the device body 3.

The indwelling device 1 according to the first embodiment includes the device body 3 and the engaging part 5 connected to the device body 3 and capable of abutting on the bodily wall 99. Since the engaging part 5 can abut on the bodily wall 99 in a state that the region of the engaging part 15 excluding the end parts 5a and 5b is expanded larger than the outer diameter of the device body 3, the indwelling device 1 can be securely placed in the body without occluding the inside of the body and damaging the patient's bodily wall.

### (Second Embodiment)

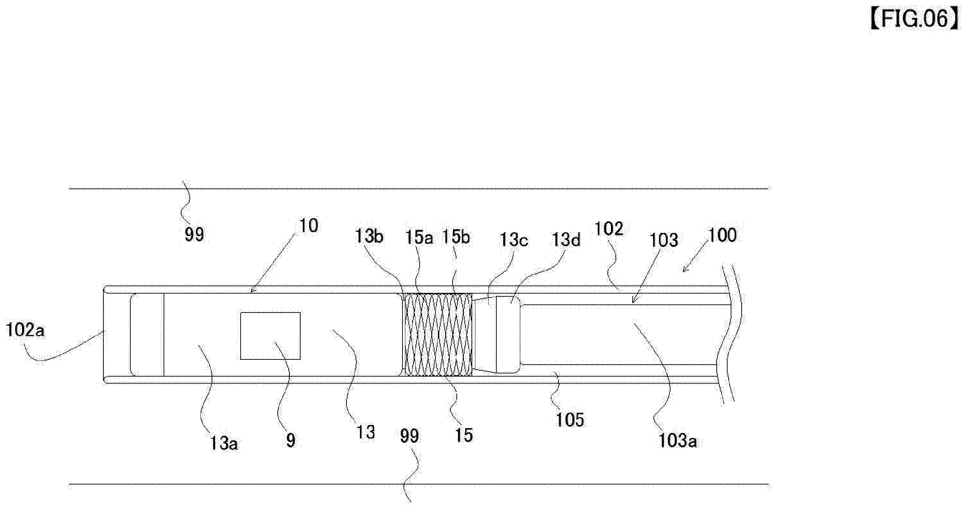

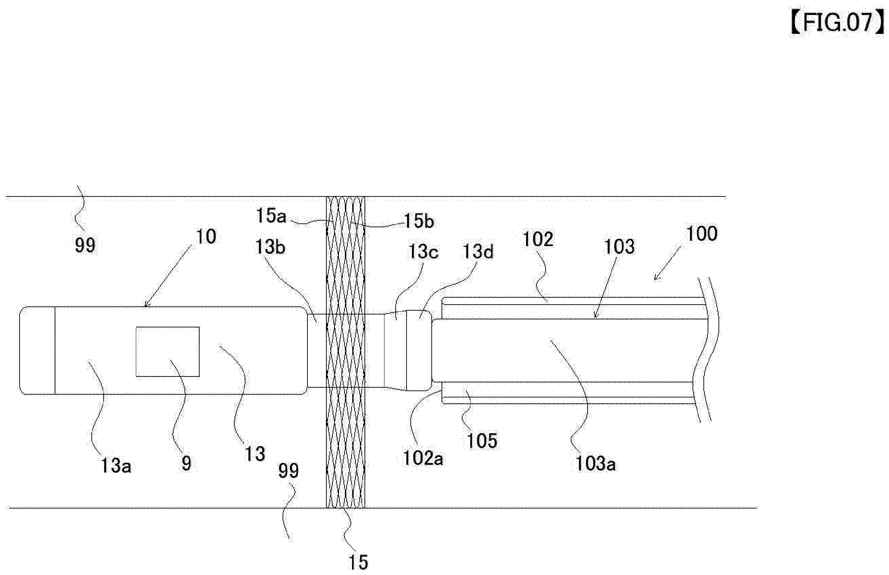

Next, the second embodiment of the present invention will be explained. FIG. 6 is an enlarged sectional view corresponding to FIG. 4 in a case that an indwelling device according to the second embodiment is disposed. FIG. 7 is an enlarged sectional view corresponding to FIG. 5 in a case that the indwelling device according to the second embodiment is disposed.

In the second embodiment, the same members as in the first embodiment are given the same reference numerals as in the first embodiment, and the explanation of the same members is omitted. In addition, a medical appliance for inserting an indwelling device 10 according to the second embodiment into a patient's body is the same as the medical appliance 100.

Also in the second embodiment, the indwelling device 10 is explained in such a way that the advancing direction of the medical appliance 100 i.e. a direction toward a patient's lesion is a distal end side and an opposite side thereto is a proximal end side.

As illustrated in FIG. 6, the indwelling device 10 includes the IC tag 9 that stores various information in the inside thereof. The indwelling device 10 is composed of a device body 13, and an engaging part 15 connected to the proximal end side of the device body 13.

Like the device body 3 according to the first embodiment, the device body 13 according to the second embodiment is also a hollow cylindrical member having a diameter of several millimeters. As a material for the device body 13, a resin material made of a biocompatible material, e.g. various synthetic resin materials including a polyolefin resin such as a polyamide resin, a polyamide elastomer, a polyester, a polyurethane, a polyvinyl chloride resin, a polyethylene, a polypropylene and an ethylene-propylene copolymer, a fluororesin such as Teflon (registered trademark), an ethylene-vinyl acetate copolymer, and the like; as well as a metal material made of a biocompatible material can be used.

The engaging part 15 according to the second embodiment is composed of a braid obtained by braiding a plurality of wires 15a wound in one direction and a plurality of wires 15b wound in a direction intersecting with the winding direction of the plurality of wires 15a. The engaging part 15 is wound around a proximal outer periphery of the device body 13 in a state that the braid is contracted. The plurality of the wires 15a and the plurality of the wires 15b are made of e.g. an NiTi shape-memory alloy.

Incidentally, the device body 13 according to the second embodiment is composed of a distal end part 13a, a small-diameter part 13b having an outer diameter smaller than of the distal end part 13a, a tapered part 13c located on the proximal end side of the small-diameter part 13b and having a diameter gradually increasing toward the proximal direction, and a proximal end part 13d located on the proximal end side of the tapered part 13c and having an outer diameter larger than of the small-diameter part 13b. In addition, the engaging part 15 composed of the braid is stored on the small-diameter part 13b.

The indwelling device 10 explained above is inserted into the catheter lumen 105 on the distal end of the catheter body 102, and the distal end of the pusher member 103 is abutted on the proximal end of the indwelling device 10. Then, the catheter body 102 in that state is inserted into the patient's body and advanced to the vicinity of the patient's lesion. FIG. 6 illustrates that state.

Then, when the grip part body 106b is turned in the R1 direction with respect to the connector 104, the grip part 106 moves to the left side of the figure with respect to the connector 104, and at the same time, the pusher member 103 also moves to the left side of the figure, the indwelling device 10 is pushed out from the catheter body 102 as illustrated in FIG. 7, and the engaging part 15 composed of the braid is expanded, so that the engaging part 15 abuts on and engages with the bodily wall 99 in a state that a region of the engaging part 15 excluding the end parts is expanded larger than the outer diameter of the device body 13.

The indwelling device 10 according to the second embodiment includes the device body 13 and the engaging part 15 connected to the device body 13 and capable of abutting on the bodily wall 99. Since the engaging part 15 can abut on the bodily wall 99 in a state that the region of the engaging part 15 excluding the end parts is expanded larger than the outer diameter of the device body 13, the indwelling device 10 can be securely placed in the body without occluding the inside of the body and damaging the patient's bodily wall 99.

Additionally, according to the indwelling device 10 of the second embodiment, the device body 13 includes a small-diameter part 13b having an outer diameter smaller than of other regions, and the engaging part 15 is stored on the small-diameter part 13b, so that the indwelling device 10 becomes easier to accommodate in the catheter lumen 105 of the catheter body 102, and deliverability of the indwelling device 10 can be improved.

### (Third Embodiment)

Next, the third embodiment of the present invention will be explained. FIG. 8 is an overall view of an indwelling device according to the third embodiment.

Incidentally, a medical appliance for inserting an indwelling device 20 according to the third embodiment into a patient's body is the same as the medical appliance 100. Also in the third embodiment, the indwelling device 20 is explained in such a way that an advancing direction of the medical appliance 100 i.e. a direction toward a patient's lesion is a distal end side and an opposite side thereto is a proximal end side.

As illustrated in FIG. 8, the indwelling device 20 includes the IC tag 9 that stores various information in the inside thereof. The indwelling device 20 is composed of a device body 23, and an engaging part 25 connected to the proximal end side of the device body 23.

The device body 23 according to the third embodiment is composed of a distal end part 23a, a small-diameter part 23b having an outer diameter smaller than of the distal end part 23a, a tapered part 23c located on the proximal end side of the small-diameter part 23b and having a diameter gradually increasing toward the proximal direction, and a proximal end part 23d located on the proximal end side of the tapered part 23c and having an outer diameter larger than of the small-diameter part 23b.

Incidentally, like the device body 3 according to the first embodiment, the device body 23 is also a hollow cylindrical member having a diameter of several millimeters. As a material for the device body 23, a resin material made of a biocompatible material, e.g. various synthetic resin materials including a polyolefin resin such as a polyamide resin, a polyamide elastomer, a polyester, a polyurethane, a polyvinyl chloride resin, a polyethylene, a polypropylene and an ethylene-propylene copolymer, a fluororesin such as Teflon (registered trademark), an ethylene-vinyl acetate copolymer, and the like; as well as a metal material made of a biocompatible material can be used.

The engaging part 25 according to the third embodiment is composed of a coil constructed by winding one NiTi shape-memory alloy wire in a hollow shape, and the engaging part 25 is wound around a proximal outer periphery of the device body 23 in a state that the coil is contracted. Incidentally, in the engaging part 25 according to the third embodiment, a distal end part 25a of the coil is fixed to the device body, a proximal end part 25b of the coil is a free end, and the engaging part 25 is stored on the small-diameter part 23b of the device body 23.

Incidentally, since the engaging part 25 according to the third embodiment is simply constructed by winding one NiTi shape-memory alloy wire in a hollow shape, the engaging part that exerts an effect described below in the third embodiment can be easily constructed.

The indwelling device 20 explained above is inserted into the catheter lumen 105 on the distal end of the catheter body 102, and the distal end of the pusher member 103 is abutted on the proximal end of the indwelling device 20. Then, the catheter body 102 in that state is inserted into the patient's body and advanced to the vicinity of the patient's lesion.

Incidentally, as the indwelling device 20 according to the third embodiment, the whole indwelling device is not inserted into the catheter lumen 105 unlike the indwelling device 1 according to the first embodiment and the indwelling device 10 according to the second embodiment, and only the small-diameter part 23b, the tapered part 23c and the proximal end part 23d, as well as the engaging part 25 stored on the small-diameter part 23b are inserted into the catheter lumen 105.

Thus, a pushed length of the indwelling device 20 pushed out from the catheter body 102 is shortened, the engaging part 25 can be swiftly abutted on the bodily wall 99, and consequently the indwelling device 20 can be swiftly placed in the patient's body.

Then, when the grip part body 106b is turned in the R1 direction with respect to the connector 104, the grip part 106 moves to the left side of the figure with respect to the connector 104, and at the same time, the pusher member 103 also moves to the left side of the figure, the indwelling device 20 is pushed out from the catheter body 102, and the engaging part 25 composed of the coil is expanded, so that the engaging part 25 abuts on and engages with the bodily wall 99 in a state that a region of the engaging part 25 excluding the end parts is expanded larger than an outer diameter of the device body 23.

The indwelling device 20 according to the third embodiment includes the device body 23 and the engaging part 25 connected to the device body 23 and capable of abutting on the bodily wall 99. Since the engaging part 25 can abut on the bodily wall 99 in a state that the region of the engaging part 25 excluding the end parts is expanded larger than the outer diameter of the device body 23, the indwelling device 20 can be securely placed in the body without occluding the inside of the body and damaging the patient's bodily wall 99.

Additionally, according to the indwelling device 20 of the third embodiment, the device body 23 includes the small-diameter part 23b having the outer diameter smaller than of other regions, and the engaging part 25 is stored on the small-diameter part 23b, so that the indwelling device 20 becomes easier to accommodate in the catheter lumen 105 of the catheter body 102, and deliverability of the indwelling device 20 can be improved.

### (Fourth Embodiment)

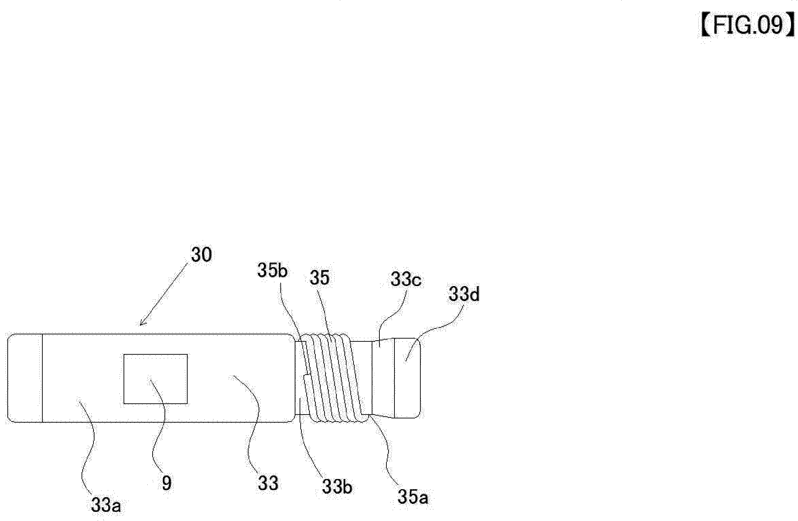

Next, the fourth embodiment of the present invention will be explained. FIG. 9 is an overall view of an indwelling device according to the fourth embodiment.

Incidentally, a medical appliance for inserting an indwelling device 30 according to the fourth embodiment into a patient's body is the same as the medical appliance 100. Also in the fourth embodiment, the indwelling device 30 is explained in such a way that the advancing direction of the medical appliance 100 i.e. a direction toward a patient's lesion is a distal end side and an opposite side thereto is a proximal end side.

As illustrated in FIG. 9, the indwelling device 30 includes the IC tag 9 that stores various information in the inside thereof. The indwelling device 30 is composed of a device body 33, and an engaging part 35 connected to the proximal end side of the device body 33.

The device body 33 according to the fourth embodiment is composed of a distal end part 33a, a small-diameter part 33b having an outer diameter smaller than of the distal end part 33a, a tapered part 33c located on the proximal end side of the small-diameter part 33b and having a diameter gradually increasing toward the proximal direction, and a proximal end part 33d located on the proximal end side of the tapered part 33c and having an outer diameter larger than of the small-diameter part 33b.

Incidentally, like the device body 3 according to the first embodiment, the device body 33 is also a hollow cylindrical member having a diameter of several millimeters. As a material for the device body 33, a resin material made of a biocompatible material, e.g. various synthetic resin materials including a polyolefin resin such as a polyamide resin, a polyamide elastomer, a polyester, a polyurethane, a polyvinyl chloride resin, a polyethylene, a polypropylene and an ethylene-propylene copolymer, a fluororesin such as Teflon (registered trademark), an ethylene-vinyl acetate copolymer, and the like; as well as a metal material made of a biocompatible material can be used.

The engaging part 35 according to the fourth embodiment is composed of a coil constructed by winding one NiTi shape-memory alloy wire in a hollow shape, and the engaging part 35 is wound around a proximal outer periphery of the device body 33 in a state that the coil is contracted. Incidentally, unlike the engaging part 25 according to the third embodiment, in the engaging part 35 according to the fourth embodiment, a proximal end part 35a of the coil is fixed to the device body 33, a distal end part 35b of the coil is a free end, and the engaging part 35 is stored on the small-diameter part 33b of the device body 33.

That means, in the engaging part 35 according to the fourth embodiment, since the end part 35b on the side not connected to the device body 33 is located on the distal end side in an advancing direction of the indwelling device 30 while being placed in the body, the engaging part 35 can be swiftly abutted on the bodily wall 99, and consequently the indwelling device 30 can be swiftly placed in the patient's body.

In addition, since the engaging part 35 according to the fourth embodiment is simply constructed by winding one NiTi shape-memory alloy wire in a hollow shape, the engaging part that exerts an effect described below in the fourth embodiment can be easily constructed.

The indwelling device 30 explained above is inserted into the catheter lumen 105 on the distal end of the catheter body 102, and the distal end of the pusher member 103 is abutted on the proximal end of the indwelling device 30. Then, the catheter body 102 in that state is inserted into the patient's body and advanced to the vicinity of the patient's lesion.

Then, when the grip part body 106b is turned in the R1 direction with respect to the connector 104, the grip part 106 moves to the left side of the figure with respect to the connector 104, and at the same time, the pusher member 103 also moves to the left side of the figure, the indwelling device 30 is pushed out from the catheter body 102, and the engaging part 35 composed of the coil is expanded, so that the engaging part 35 abuts on and engages with the bodily wall 99 in a state that a region of the engaging part 35 excluding the end parts is expanded larger than an outer diameter of the device body 33.

The indwelling device 30 according to the fourth embodiment includes the device body 33 and the engaging part 35 connected to the device body 33 and capable of abutting on the bodily wall 99. Since the engaging part 35 can abut on the bodily wall 99 in a state that the region of the engaging part 35 excluding the end parts is expanded larger than the outer diameter of the device body 33, the indwelling device 30 can be securely placed in the body without occluding the inside of the body and damaging the patient's bodily wall 99.

Additionally, according to the indwelling device 30 of the fourth embodiment, the device body 33 includes the small-diameter part 33b having the outer diameter smaller than of other regions, and the engaging part 35 is stored on the small-diameter part 33b, so that the indwelling device 30 becomes easier to accommodate in the catheter lumen 105 of the catheter body 102, and deliverability of the indwelling device 30 can be improved.

Furthermore, according to the indwelling device 30 of the fourth embodiment, in the coil of the engaging part 35, the end part 35b on the side not connected to the device body 33 is located closer to a distal end side in an advancing direction of the indwelling device 30 while being placed in the body than the end part 35a on the side connected to the device body 33, so that the engaging part 35 can be swiftly abutted on the bodily wall 99, and consequently the indwelling device 30 can be swiftly placed in the body.

### (Fifth Embodiment)

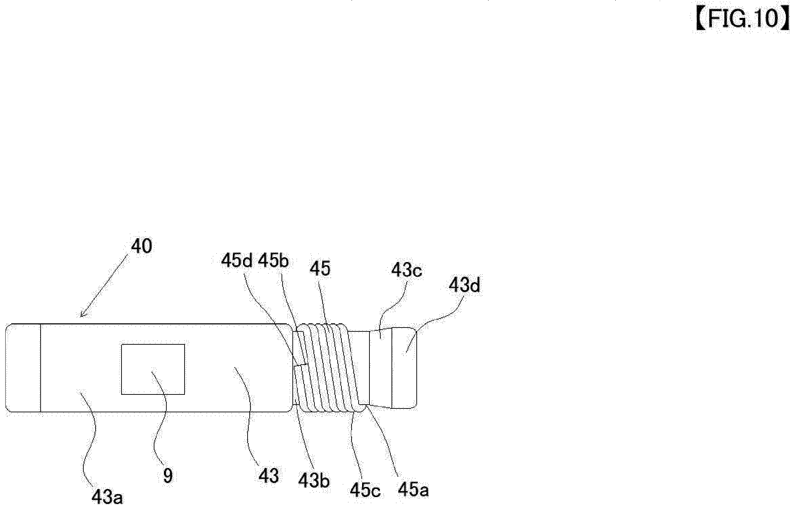

Next, the fourth embodiment of the present invention will be explained. FIG. 10 is an overall view of an indwelling device according to the fourth embodiment.

Incidentally, a medical appliance for inserting an indwelling device 40 according to the fifth embodiment into a patient's body is the same as the medical appliance 100. Also in the fifth embodiment, the indwelling device 40 is explained in such a way that the advancing direction of the medical appliance 100 i.e. a direction toward a patient's lesion is a distal end side and an opposite side thereto is a proximal end side.

As illustrated in FIG. 10, the indwelling device 40 includes the IC tag 9 that stores various information in the inside thereof. The indwelling device 40 is composed of a device body 43, and an engaging part 45 connected to the proximal end side of the device body 43.

The device body 43 according to the fifth embodiment is composed of a distal end part 43a, a small-diameter part 43b having an outer diameter smaller than of the distal end part 43a, a tapered part 43c located on the proximal end side of the small-diameter part 43b and having a diameter gradually increasing toward the proximal direction, and a proximal end part 43d located on the proximal end side of the tapered part 43c and having an outer diameter larger than of the small-diameter part 43b.

Incidentally, like the device body 3 according to the first embodiment, the device body 43 is also a hollow cylindrical member having a diameter of several millimeters. As a material for the device body 43, a resin material made of a biocompatible material, e.g. various synthetic resin materials including a polyolefin resin such as a polyamide resin, a polyamide elastomer, a polyester, a polyurethane, a polyvinyl chloride resin, a polyethylene, a polypropylene and an ethylene-propylene copolymer, a fluororesin such as Teflon (registered trademark), an ethylene-vinyl acetate copolymer, and the like; as well as a metal material made of a biocompatible material can be used.

Unlike the engaging part 35 according to the fourth embodiment, the engaging part 45 according to the fifth embodiment is composed of a coil constructed by winding two NiTi shape-memory alloy wires in a hollow shape, and the engaging part 45 is wound around a proximal outer periphery of the device body 43 in a state that the coil is contracted. Incidentally, like the engaging part 35 according to the fourth embodiment, also in the engaging part 45 according to the fifth embodiment, a proximal end part 45a and a proximal end part 45c of the coil are fixed to the device body 43, a distal end part 45b and a distal end part 45d of the coil are free ends, and the engaging part 45 is stored on the small-diameter part 43b of the device body 43.

Incidentally, since the engaging part 45 according to the fifth embodiment is simply constructed by winding two NiTi shape-memory alloy wires in a hollow shape, the engaging part that exerts an effect described below in the fifth embodiment can be easily constructed.

The indwelling device 40 explained above is inserted into the catheter lumen 105 on the distal end of the catheter body 102, and the distal end of the pusher member 103 is abutted on the proximal end of the indwelling device 40. Then, the catheter body 102 in that state is inserted into the patient's body and advanced to the vicinity of the patient's lesion.

Then, when the grip part body 106b is turned in the R1 direction with respect to the connector 104, the grip part 106 moves to the left side of the figure with respect to the connector 104, and at the same time, the pusher member 103 also moves to the left side of the figure, the indwelling device 40 is pushed out from the catheter body 102, and the engaging part 45 composed of the coil is expanded, so that the engaging part 45 abuts on and engages with the bodily wall 99 in a state that a region of the engaging part 45 excluding the end parts is expanded larger than an outer diameter of the device body 43.

The indwelling device 40 according to the fifth embodiment includes the device body 43 and the engaging part 45 connected to the device body 43 and capable of abutting on the bodily wall 99. Since the engaging part 45 can abut on the bodily wall 99 in a state that the region of the engaging part 45 excluding the end parts is expanded larger than the outer diameter of the device body 43, the indwelling device 40 can be securely placed in the body without occluding the inside of the body and damaging the patient's bodily wall 99.

Additionally, according to the indwelling device 40 of the fifth embodiment, the device body 43 includes the small-diameter part 43b having the outer diameter smaller than of other regions, and the engaging part 45 is stored on the small-diameter part 43b, so that the indwelling device 30 becomes easier to accommodate in the catheter lumen 105 of the catheter body 102, and deliverability of the indwelling device 40 can be improved.

Additionally, according to the indwelling device 40 of the fifth embodiment, in the coil of the engaging part 45, the end part 45b on the side not connected to the device body 43 is located closer to a distal end side in an advancing direction of the indwelling device 40 while being placed in the body than the end part 45a on the side connected to the device body 43, so that the engaging part 45 can be swiftly abutted on the bodily wall 99, and consequently the indwelling device 40 can be swiftly placed in the body.

Furthermore, according to the indwelling device 40 of the fifth embodiment, since the coil as the engaging part 45 is composed of two wires, the indwelling device 40 can be more firmly placed in the bodily wall 99.

Incidentally, in the fifth embodiment, although the engaging part 45 is formed as the coil composed of two NiTi shape-memory alloy wires, the coil may be composed of two or more NiTi shape-memory alloy wires, and the coil as the engaging part 45 may be composed of a wire made of a biocompatible shape-memory alloy other than the NiTi shape-memory alloy.

### (Sixth Embodiment)

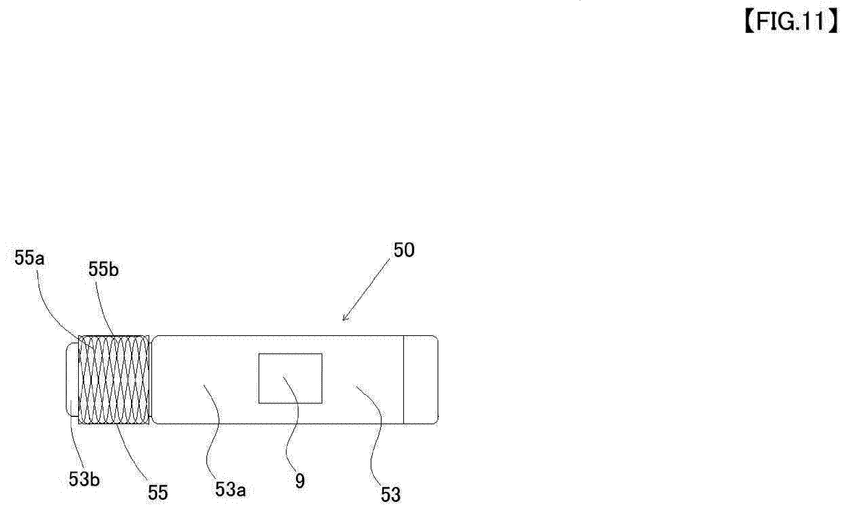

Next, the sixth embodiment of the present invention will be explained. FIG. 11 is an overall view of an indwelling device according to the sixth embodiment.

Incidentally, a medical appliance for inserting an indwelling device 50 according to the sixth embodiment into a patient's body is the same as the medical appliance 100. Also in the sixth embodiment, the indwelling device 50 is explained in such a way that the advancing direction of the medical appliance 100 i.e. a direction toward a patient's lesion is a distal end side and an opposite side thereto is a proximal end side.

As illustrated in FIG. 11, the indwelling device 50 includes the IC tag 9 that stores various information in the inside thereof, and the indwelling device 50 is composed of a device body 53 and an engaging part 55 connected to the distal end side of the device body 53. That means, unlike the device bodies according to the first to fifth embodiments, the device body 53 according to the sixth embodiment has a configuration in which the engaging part 55 is disposed on the distal end side of the device body 53.

In addition, the device body 53 is composed of a small-diameter part 53b on the distal end side and a proximal end part 53a having an outer diameter larger than of the small-diameter part 53b.

Incidentally, like the device body 3 according to the first embodiment, the device body 53 is also a hollow cylindrical member having a diameter of several millimeters. As a material for the device body 53, a resin material made of a biocompatible material, e.g. various synthetic resin materials including a polyolefin resin such as a polyamide resin, a polyamide elastomer, a polyester, a polyurethane, a polyvinyl chloride resin, a polyethylene, a polypropylene and an ethylene-propylene copolymer, a fluororesin such as Teflon (registered trademark), an ethylene-vinyl acetate copolymer, and the like; as well as a metal material made of a biocompatible material can be used.

Like the engaging part 15 according to the second embodiment, the engaging part 55 according to the sixth embodiment is composed of a braid obtained by braiding a plurality of wires 55a wound in one direction and a plurality of wires 55b wound in a direction intersecting with the winding direction of the plurality of wires 55a. The engaging part 55 is stored on the small-diameter part 53b of the device body 53 in a state that the braid is contracted. The plurality of the wires 55a and the plurality of the wires 55b are made of e.g. an NiTi shape-memory alloy.

The indwelling device 50 explained above is inserted into the catheter lumen 105 on the distal end of the catheter body 102, and the distal end of the pusher member 103 is abutted on the proximal end of the indwelling device 50. Then, the catheter body 102 in that state is inserted into the patient's body and advanced to the vicinity of the patient's lesion.

Then, when the grip part body 106b is turned in the R1 direction with respect to the connector 104, the grip part 106 moves to the left side of the figure with respect to the connector 104, and at the same time, the pusher member 103 also moves to the left side of the figure, the indwelling device 50 is pushed out from the catheter body 102, and the engaging part 55 composed of the coil is expanded, so that the engaging part 55 abuts on and engages with the bodily wall 99 in a state that a region of the engaging part 55 excluding the end parts is expanded larger than an outer diameter of the device body 53.

The indwelling device 50 according to the sixth embodiment includes the device body 53 and the engaging part 55 connected to the device body 53 and capable of abutting on the bodily wall 99. Since the engaging part 55 can abut on the bodily wall 99 in a state that the region of the engaging part 55 excluding the end parts is expanded larger than the outer diameter of the device body 53, the indwelling device 50 can be securely placed in the body without occluding the inside of the body and damaging the patient's bodily wall 99.

Additionally, according to the indwelling device 50 of the sixth embodiment, the device body 53 includes the small-diameter part 53b having the outer diameter smaller than of other regions, and the engaging part 55 is wound around the small-diameter part 53b, so that the indwelling device 50 becomes easier to accommodate in the catheter lumen 105 of the catheter body 102, and deliverability of the indwelling device 50 can be improved.

Furthermore, according to the indwelling device 50 of the sixth embodiment, since the engaging part 55 is disposed on the distal end side of the device body 53, the engaging part 55 can be swiftly abutted on the bodily wall 99 before the whole indwelling device 50 is pushed out from the catheter body 102, and consequently the indwelling device 50 can be swiftly placed in the body.

### (Seventh Embodiment)

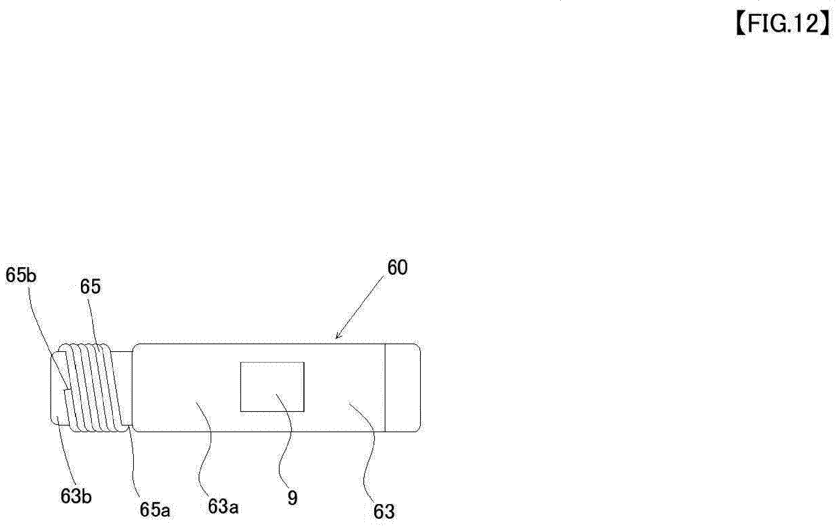

Finally, the seventh embodiment of the present invention will be explained. FIG. 12 is an overall view of an indwelling device according to the seventh embodiment.

Incidentally, a medical appliance for inserting an indwelling device 60 according to the seventh embodiment into a patient's body is the same as the medical appliance 100. Also in the seventh embodiment, the indwelling device 60 is explained in such a way that the advancing direction of the medical appliance 100 i.e. a direction toward a patient's lesion is a distal end side and an opposite side thereto is a proximal end side.

As illustrated in FIG. 12, the indwelling device 60 includes the IC tag 9 that stores various information in the inside thereof. Like the indwelling device 50 according to the sixth embodiment, the indwelling device 60 is composed of a device body 63, and an engaging part 65 connected to the distal end side of the device body 63.

In addition, the device body 63 is composed of a small-diameter part 63b on the distal end side and a proximal end part 63a having an outer diameter larger than of the small-diameter part 63b.

Incidentally, like the device body 3 according to the first embodiment, the device body 63 is also a hollow cylindrical member having a diameter of several millimeters. As a material for the device body 63, a resin material made of a biocompatible material, e.g. various synthetic resin materials including a polyolefin resin such as a polyamide resin, a polyamide elastomer, a polyester, a polyurethane, a polyvinyl chloride resin, a polyethylene, a polypropylene and an ethylene-propylene copolymer, a fluororesin such as Teflon (registered trademark), an ethylene-vinyl acetate copolymer, and the like; as well as a metal material made of a biocompatible material can be used.

The engaging part 65 according to the seventh embodiment is composed of a coil constructed by winding one NiTi shape-memory alloy wire in a hollow shape, and the engaging part 65 is wound around an outer periphery of the small-diameter part 63b of the device body 63 in a state that the coil is contracted. Incidentally, in the engaging part 65 according to the seventh embodiment, a proximal end part 65a of the coil is fixed to the device body, and a distal end part 65b of the coil is a free end.

That means, in the engaging part 65 according to the seventh embodiment, since the end part 65b on the side not connected to the device body 63 is located on the distal end side in an advancing direction of the indwelling device 60 while being placed in the body, the engaging part 65 can be swiftly abutted on the bodily wall 99, and consequently the indwelling device 30 can be swiftly placed in the patient's body.

In addition, since the engaging part 65 according to the seventh embodiment is simply constructed by winding one NiTi shape-memory alloy wire in a hollow shape, the engaging part that exerts an effect described below in the seventh embodiment can be easily constructed.

The indwelling device 60 explained above is inserted into the catheter lumen 105 on the distal end of the catheter body 102, and the distal end of the pusher member 103 is abutted on the proximal end of the indwelling device 60. Then, the catheter body 102 in that state is inserted into the patient's body and advanced to the vicinity of the patient's lesion.

Then, when the grip part body 106b is turned in the R1 direction with respect to the connector 104, the grip part 106 moves to the left side of the figure with respect to the connector 104, and at the same time, the pusher member 103 also moves to the left side of the figure, the indwelling device 60 is pushed out from the catheter body 102, and the engaging part 65 composed of the coil is expanded, so that the engaging part 65 abuts on and engages with the bodily wall 99 in a state that a region of the engaging part 36 excluding the end parts is expanded larger than an outer diameter of the device body 63.

The indwelling device 60 according to the seventh embodiment includes the device body 63 and the engaging part 65 connected to the device body 63 and capable of abutting on the bodily wall 99. Since the engaging part 65 can abut on the bodily wall 99 in a state that the region of the engaging part 65 excluding the end parts is expanded larger than the outer diameter of the device body 63, the indwelling device 60 can be securely placed in the body without occluding the inside of the body and damaging the patient's bodily wall 99.

Additionally, according to the indwelling device 60 of the seventh embodiment, the device body 63 includes the small-diameter part 63b having the outer diameter smaller than of other regions, and the engaging part 65 is wound around the small-diameter part 63b, so that the indwelling device 60 becomes easier to accommodate in the catheter lumen 105 of the catheter body 102, and deliverability of the indwelling device 60 can be improved.

Furthermore, according to the indwelling device 60 of the seventh embodiment, the engaging part 65 is disposed on the distal end side of the device body 63, and in the coil of the engaging part 65, the end part 65b on the side not connected to the device body 63 is located closer to a distal end side in an advancing direction of the indwelling device 60 while being placed in the body than the end part 65a on the side connected to the device body 63, so that the engaging part 65 can be more swiftly abutted on the bodily wall 99, and consequently the indwelling device 60 can be more swiftly placed in the body.

The indwelling devices according to various embodiments of the present invention have been explained above, however, the present invention is not limited to the above embodiments, and can be variously modified and executed without departing from the gist of the invention.

For example, the engaging part 5 according to the first embodiment and the engaging part 45 according to the fifth embodiment may be connected to the small-diameter part 63b of the device body 63 according to the seventh embodiment. Thereby, the engaging part 5 and the engaging part 45 can be swiftly abutted on the bodily wall 99, and consequently the indwelling device can be swiftly placed in the body.

On the other hand, the engaging part 15 according to the second embodiment, the engaging part 35 according to the fourth embodiment, and the engaging part 45 according to the fifth embodiment can also be connected to the proximal end side of the device body 3 according to the first embodiment.

### DESCRIPTION OF REFERENCE NUMERALS

- 1, 10, 20, 30, 40, 50, 60: Indwelling device
- 3, 13, 23, 33, 43, 53, 63: Device body
- 5, 15, 25, 35, 45, 55, 65: Engaging part
- 9: IC tag
- 13b, 23b, 33b, 43b, 53b, 63b: Small-diameter part
- 99: Bodily wall
- 100: Medical appliance
- 102: Catheter body
- 103: Pusher member
- 104: Connector
- 105: Catheter lumen
- 106: Grip part

## Claims

1. An indwelling device comprising
a device body and
an engaging part connected to the device body and capable of abutting on a bodily wall, wherein
the engaging part can abut on the bodily wall in a state that a region of the engaging part, excluding end parts, is expanded larger than an outer diameter of the device body.

2. The indwelling device according to claim 1, wherein
the device body comprises a small-diameter part having an outer diameter smaller than of other regions, and
the engaging part can be stored on the small-diameter part.

3. The indwelling device according to claim 1 or 2, wherein the engaging part is constructed by spirally winding one or more wires.

4. The indwelling device according to claim 3, wherein, in the wire, an end part on a side not connected to the device body is located on a distal end side in an advancing direction of the indwelling device while being placed in a body.
